# FASCICULE DE BREVET EUROPEEN

(11) **EP 2 135 585 B1**
(45) Date de publication et mention de la délivrance du brevet: **23.07.2014**
(21) Numéro de dépôt: 09162279.5
(22) Date de dépôt: 09.06.2009
(51) Int. Cl.: A61F 2/40

(54) **Prothèse d'épaule inversée**
Inverse Schulterprothese
Inverted shoulder prosthesis

(30) Priorité: 17.06.2008 FR 0853984
(43) Date de publication de la demande: 23.12.2009
(73) Titulaire: Euros, 13600 La Ciotat (FR)
(72) Inventeur: Odella, Ferdinando, I-20122, MILAN (IT); Gravier, Renaud, 13008, Marseille (FR); Gadea, José, 83200, Toulon (FR); Simonet, Jean-Yves, 66750, Saint-Cyprien Plage (FR); Clement, Thierry, 13009, Marseille (FR)
(74) Mandataire: Lemoine, Jean-Sébastien

(56) Documents cités:
- EP-A- 0 299 889
- EP-A- 1 402 854
- EP-A- 1 591 084
- EP-A- 1 607 070
- WO-A-2007/039820
- WO-A-2008/015724
- DE-A1-102006 041 550
- US-A- 5 462 563
- US-A1- 2004 220 673
- US-B1- 6 790 234

## Description

La présente invention concerne une prothèse d'épaule inversée, c'est-à-dire une prothèse d'épaule comportant un implant huméral présentant une surface de réception pour une sphère portée par un implant glénoïdien correspondant.

Contrairement à la prothèse d'épaule naturelle (sphère portée par l'implant, huméral, reproduisant ainsi la structure anatomique), la prothèse d'épaule inversée permet de palier à la perte de stabilité de l'articulation gléno-humérale causée par la déficience de la coiffe des rotateurs. Cet effet est obtenu en décalant le centre de rotation vers le corps du patient et en l'immobilisant par la congruence entre la sphère glénoïdienne et la surface de réception humérale.

Dans les prosthèses d'épaule inversées connues, l'implant huméral possède un angle cervico-diaphysaire égal à 155° ou 145°, selon les conceptions. Ainsi la demande de brevet US 2004/220673. divulgue une telle prothèse avec un angle cervico-diaphysaire égal à environ 150° l'axe de symétrie de la surface hémisphérique de la sphère glénoïdienne étant excentré par rapport l'organe de fixation.

Or, toutes ces prothèses présentent comme problème un conflit entre l'implant humérale et la partie inférieure de la glène osseuse lors des mouvements d'adduction, ce qui provoque une douleur chez le patient, voire un descellement de l'embase glénoïdienne à laquelle est fixée la sphère.

L'invention vise à réaliser une prothèse d'épaule inversée ne présentant pas l'inconvénient précité.

Selon l'invention, l'implant huméral comporte un angle cervico-diaphysaire égal à 135°.

Ainsi, lors d'un mouvement d'adduction, quand le bras est le long du corps, d'une part, le plan défini par l'extrémité supérieure de la paroi délimitant la surface de réception humérale a une orientation plus verticale que dans les prothèses d'épaule inversées traditionnelles, et, d'autre part, la position de l'axe de la sphère est abaissée par rapport à l'omoplate en comparaison avec les mêmes prothèses d'épaule inversées, ce qui limite fortement, voire annule, le conflit entre l'implant huméral avec la glène osseuse.

D'autres particularités et avantages de la présente invention apparaîtront dans la description d'un mode de réalisation donné à titre d'exemple non limitatif et illustré par les dessins mis en annexe dans lesquels :
La figure 1 est une vue schématique d'une prothèse d'épaule inversée en adduction, pour une prothèse de l'art antérieur ayant un angle cervico-diaphysaire égal à 155° ;
La figure 2 est une vue similaire à la figure 1, la prothèse, conforme à la présente invention, ayant un angle cervico-diaphysaire égal à 135° ;
La figure 3 est une vue similaire à la figure 2, la prothèse conforme à la présente invention étant en abduction ;
La figure 4 est une vue en perspective éclatée de la prothèse conforme à la présente invention ;
La figure 5 est une vue en coupe schématique agrandie de la fixation de l'insert huméral portant la surface de réception à la tige humérale ;
La figure 6 est une vue en coupe de l'insert huméral ; et
La figure 7 est une vue en coupe de l'implant glénoïdien de la prothèse d'épaule inversée conforme à la présente invention.

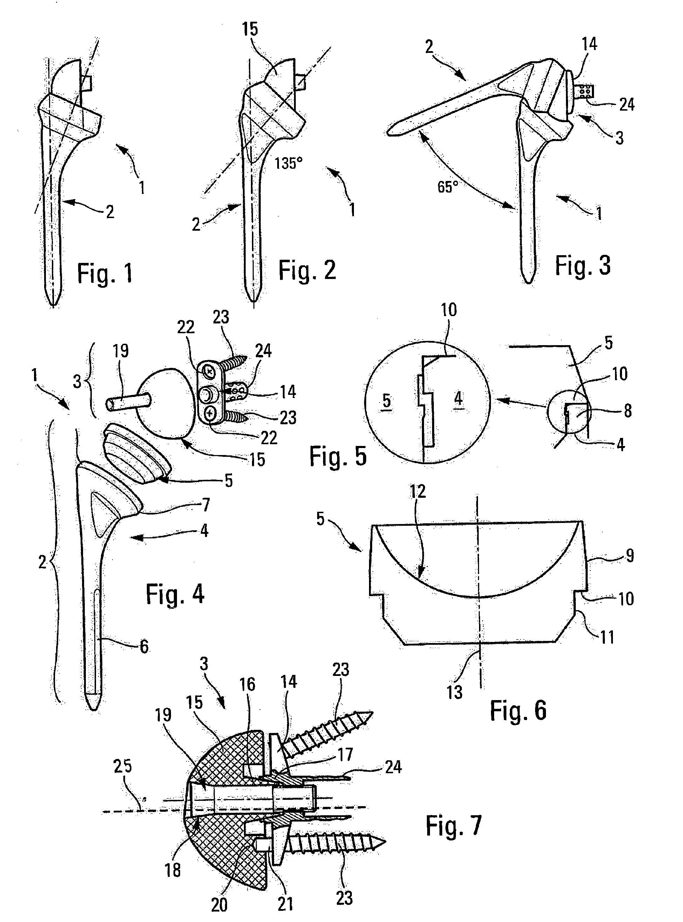

De façon classique, une prothèse d'épaule inversée 1 comprend un implant huméral 2 et un implant glénoïdien 3. Selon l'invention, l'angle cervico-diaphysaire de l'implant huméral 2 est égal à 135°. Cet angle, plus faible que les angles des prothèses d'épaule inversées connues, permet de réduire considérablement le conflit existant entre la glène osseuse de l'implant huméral 2 quand le bras est en adduction. Il n'y a pas d'augmentation significative du conflit existant entre la glène osseuse de l'implant huméral 2 quand le bras est en abduction du fait, qu'en abduction, la rotation du bras est limitée à 65° du fait de la présence du ligament gléno-huméral (cf. figure 3).

Comme on peut le voir à la figure 4, dans le présent mode de réalisation, l'implant huméral 2 comprend une tige humérale 4 qui est destinée à être fixée à l'humérus, et un insert huméral 5 qui est adapté à être fixé à la tige humérale 4 et qui est destinée à recevoir, en articulation, l'implant glénoïdien 3.

De façon plus précise, la tige humérale 4 possède une quille diaphysaire 6 qui s'étend selon une direction longitudinale et est adaptée à être introduite dans le fût de l'humérus, et une tête épiphysaire 7 auquel l'insert huméral 5 est fixé. La tête épiphysaire 7 comprend une paroi cylindrique 8 continue dont l'extrémité supérieure définit un plan dont la normale fait un angle de 135° avec la quille diaphysaire 6 (cf. figure 2).

L'insert huméral 5 comprend une paroi externe cylindrique 9, qui en l'occurrence, prolonge la paroi cylindrique 8 de la tête épiphysaire 7, et qui comprend, à son extrémité inférieure, un épaulement annulaire 10 par lequel l'insert huméral 5 vient en butée contre l'extrémité supérieure de la paroi cylindrique 8 de la tête épihysaire 7. En l'occurrence, l'insert huméral 5 est fixé à la tige humérale 4 par encliquetage entre la surface interne de la paroi cylindrique 8 de la tête épiphysaire 7 et une surface cylindrique de fixation 11 de l'insert 5 située sous l'épaulement annulaire 10.

En outre, l'insert huméral 5, de symétrie axiale, comprend une surface de réception 12 de l'implant glénoïdien 3, qui est une surface en forme de calotte sphérique. Dans le présent mode de réalisation, la calotte sphérique présente une ouverture supérieure circulaire dans le plan défini par l'extrémité libre de la paroi externe cylindrique 9 de l'insert huméral 5 dont le rayon est le double de la profondeur ce cette calotte prise dans l'axe de symétrie 13 de l'insert 5.

Par ailleurs, l'implant glénoïdien 3 possède une embase 14 adaptée à être fixée à l'omoplate, et une sphère glénoïdienne 15 fixée à l'embase 14 et réalisant l'articulation avec l'implant huméral 2 (de façon plus précise, avec l'insert huméral 5).

La sphère glénoïdienne 15 comprend, à sa face avant, une surface hémisphérique par laquelle est réalisée l'articulation avec la surface de réception 12 de l'insert huméral 5, les deux surfaces ayant des formes congrues, et, à sa face arrière, une surface plane conformée de façon à permettre sa fixation à l'embase 14. La surface plane arrière comprend un organe de fixation (en l'occurrence, un cône male de fixation 16) venant en prise avec un organe complémentaire de fixation porté par l'embase 14 (en l'occurrence, un cône femelle 17 dans lequel le cône mâle 16 vient en prise). Pour réaliser la fixation de la sphère 15 à l'embase 14, la première comporte un orifice 18 qui la traverse et s'étend dans le cône mâle de fixation 16. Cet orifice 18 est adapté recevoir une vis 19, le cône femelle 17 comprenant, dans le prolongement de sa section de réception du cône mâle 16, un taraudage adapté à coopérer avec le filetage de la vis 19.

Toujours dans le but de réduire de façon significative le conflit existant entre la glène osseuse de l'implant huméral 2 quand le bras est en adduction, dans la sphère glénoïdienne 15, le cône mâle 16 est excentré par rapport à l'axe de symétrie 25 de la surface hémisphérique. En outre, la sphère glénoïdienne 15 et l'embase 14 comprennent des organes d'indexation 20, 21 de sorte que, une fois l'embase 14 implantée et la sphère 15 fixée à l'embase 14, l'axe de symétrie 25 est décalée vers le bas. Dans le présent mode de réalisation, l'organe d'indexation 20 porté par la sphère 15 est un orifice 20 réalisée à la surface arrière, l'organe complémentaire 21 porté par l'embase 21 étant un pion 21 se logeant dans l'orifice 20.

Par ailleurs, afin de permettre sa fixation à l'omoplate, l'embase 14 ne comprend que deux orifices 22 pour le passage de vis 23, et une quille d'ancrage 24. Les deux orifices 22 permettent l'orientation convenable des deux vis 23, et la quille d'ancrage 24 est creuse et percée favorisant ainsi la vascularisation lors de l'auto-greffe. Dans le présent mode de réalisation, le cône femelle 17 est réalisé dans la quille d'ancrage 24.

## Revendications

1. Prothèse d'épaule inversée (1) comprenant un implant huméral (2) et un implant glénoïdien (3), l'implant glénoïdien possédant une embase (14) adaptée à être fixée à l'omoplate et une sphère glénoïdienne (15) fixée à l'embase (14) par un organe de fixation (16), la surface hémisphérique de la sphère glénoïdienne (15) présentant un axe de symétrie (25) excentré par rapport l'organe de fixation (16), **caractérisée en ce que** l'implant huméral (2) comporte un angle cervico-diaphysaire qui est égal à 135°.

2. Prothèse d'épaule inversée (1) selon la revendication 1, **caractérisée en ce que** l'implant huméral (2) comprend une tige humérale (4) possédant, d'une part, une quille diaphysaire (6) adaptée à être introduite dans le fût d'un humérus et, d'autre part, une tête épiphysaire (7) qui possède une paroi cylindrique (8) continue dont l'extrémité supérieure définit un plan dont la normale fait un angle de 135° avec la quille diaphysaire (6).

3. Prothèse d'épaule inversée (1) selon la revendication 2, **caractérisée en ce que** l'implant huméral (2) comprend un insert huméral (5) adapté à être fixé à la tête épiphysaire (7) et à recevoir, en articulation, l'implant glénoïdien (3).

4. Prothèse d'épaule inversée (1) selon la revendication 3, **caractérisée en ce que** l'insert huméral (5) comprend une surface de réception (12) de l'implant glénoïdien (3), ayant la forme d'une calotte sphérique.

5. Prothèse d'épaule inversée (1) selon la revendication 3 ou 4, **caractérisée en ce que** l'insert huméral (5) comprend une paroi externe cylindrique (9) qui possède, à son extrémité inférieure, un épaulement annulaire (10) par lequel l'insert huméral (5) est fixé à la tige humérale (4).

6. Prothèse d'épaule inversée (1) selon la revendication 5, **caractérisée en ce que** la fixation de l'insert huméral (5) à la tige humérale (4) est réalisée par encliquetage entre une surface cylindrique de fixation (11) de l'insert (5) située sous l'épaulement annulaire (10) et une surface interne de la paroi cylindrique (8) de la tête épiphysaire (7).

7. Prothèse d'épaule inversée (1) selon l'une des revendications 1 à 6, **caractérisée en ce que** l'organe de fixation (16) de la sphère glénoïdienne (15) comprend un cône male de fixation (16) réalisé à surface plane arrière de cette dernière et venant se coincer dans un cône femelle (17) réalisé dans l'embase (14).

8. Prothèse d'épaule inversée (1) selon la revendication 7, **caractérisée en ce que** la surface plane arrière de la sphère glénoïdienne (15) comprend un organe d'indexation (20) adapté à coopérer avec un organe complémentaire d'indexation (21) porté par l'embase (14) de façon à définir l'orientation de l'excentration de la sphère (15).

9. Prothèse d'épaule inversée (1) selon la revendication 8, **caractérisée en ce que** l'organe d'indexation (20) est un orifice dans laquelle vient se loger un pion (21) formant l'organe complémentaire d'indexation (21).

10. Prothèse d'épaule inversée (1) selon l'une des revendications 7 à 9, **caractérisée en ce que** le cône mâle de fixation (16) est traversé par un orifice (18) adapté recevoir une vis (19), et **en ce que** le cône femelle (17) comprend, dans le prolongement de sa section de réception du cône mâle (16), un taraudage adapté à coopérer avec le filetage de la vis (19).

## Patentansprüche

1. Inverse Schulterprothese (1), umfassend ein Humerusimplantat (2) und ein Glenoidimplantat (3), wobei das Glenoidimplantat eine Sitzfläche (14), die geeignet ist, um am Schulterblatt befestigt zu werden, und eine Glenosphäre (15), die an der Sitzfläche (14) über ein Befestigungsorgan (16) befestigt ist, besitzt, wobei die halbkugelförmige Oberfläche der Glenosphäre (15) eine Symmetrieachse (25) aufweist, die im Verhältnis zu dem Befestigungsorgan (16) außermittig ist, **dadurch gekennzeichnet, dass** das Humerusimplantat (2) einen Schenkelhals-Schenkelschaft-Winkel aufweist, der 135° beträgt.

2. Inverse Schulterprothese (1) nach Anspruch 1, **dadurch gekennzeichnet, dass** das Humerusimplantat (2) einen Humerusschaft (4) umfasst, der einerseits einen diaphysären Stiel (6), der geeignet ist, um in den Schaft eines Oberarmknochens eingeführt zu werden, und andererseits einen epiphysären Kopf (7), der eine durchgehende zylindrische Wand (8) besitzt, deren oberes Ende eine Ebene definiert, deren Senkrechte mit dem diaphysären Stiel (6) einen Winkel von 135° bildet, besitzt.

3. Inverse Schulterprothese (1) nach Anspruch 2, **dadurch gekennzeichnet, dass** das Humerusimplantat (2) einen Humeruseinsatz (5) umfasst, der geeignet ist, um an dem epiphysären Kopf (7) befestigt zu werden und das Glenoidimplantat (3) gelenkig aufzunehmen.

4. Inverse Schulterprothese (1) nach Anspruch 3, **dadurch gekennzeichnet, dass** der Humeruseinsatz (5) eine Oberfläche (12) zur Aufnahme des Glenoidimplantats (3) umfasst, welche die Form einer sphärischen Kalotte aufweist.

5. Inverse Schulterprothese (1) nach Anspruch 3 oder 4, **dadurch gekennzeichnet, dass** der Humeruseinsatz (5) eine zylindrische Außenwand (9) umfasst, die an ihrem unteren Ende eine ringförmige Schulter (10) besitzt, über die der Humeruseinsatz (5) an dem Humerusschaft (4) befestigt ist.

6. Inverse Schulterprothese (1) nach Anspruch 5, **dadurch gekennzeichnet, dass** die Befestigung des Humeruseinsatzes (5) an dem Humerusschaft (4) durch Einrasten zwischen einer zylindrischen Oberfläche (11) zum Befestigen des Einsatzes (5), die sich unter der ringförmigen Schulter (10) befindet, und einer internen Oberfläche der zylindrischen Wand (8) des epiphysären Kopfes (7) erfolgt.

7. Inverse Schulterprothese (1) nach einem der Ansprüche 1 bis 6, **dadurch gekennzeichnet, dass** das Befestigungsorgan (16) der Glenosphäre (15) einen Außenbefestigungskonus (16) umfasst, der mit einer dieser gegenüber ebenen Rückfläche ausgebildet ist und in einen Innenkonus (17), der in der Sitzfläche (14) ausgebildet ist, eingeklemmt wird.

8. Inverse Schulterprothese (1) nach Anspruch 7, **dadurch gekennzeichnet, dass** die ebene Rückfläche der Glenosphäre (15) ein Indexierungsorgan (20) umfasst, das geeignet ist, um mit einem ergänzenden Indexierungsorgan (21) zusammenzuwirken, das von der Sitzfläche (14) getragen wird, um die Orientierung der Außermittigkeit der Sphäre (15) zu definieren.

9. Inverse Schulterprothese (1) nach Anspruch 8, **dadurch gekennzeichnet, dass** das Indexierungsorgan (20) eine Öffnung ist, in die ein Stift (21) aufgenommen wird, der das ergänzende Indexierungsorgan (21) bildet.

10. Inverse Schulterprothese (1) nach einem der Ansprüche 7 bis 9, **dadurch gekennzeichnet, dass** durch den Außenbefestigungskonus (16) eine Öffnung (18) geht, die geeignet ist, um eine Schraube (19) aufzunehmen, und dass der Innenkonus (17) in der Verlängerung seines Abschnitts zur Aufnahme des Außenkonus (16) ein Innengewinde umfasst, das geeignet ist, um mit dem Außengewinde der Schraube (19) zusammenzuwirken.

## Claims

1. Inverted shoulder prosthesis (1) comprising a humeral implant (2) and a glenoid implant (3), the glenoid implant having a base (14) designed to be fixed to the scapula, and a glenoid sphere (15) fixed to the base (14) by a fixing member (16), the hemispherical surface of the glenoid sphere (15) having an axis of symmetry (25) off-centered with respect to the fixing member (16), **characterized in that** the humeral implant (2) has a cervico-diaphyseal angle equal to 135°.

2. Inverted shoulder prosthesis (1) according to Claim 1, **characterized in that** the humeral implant (2) comprises a humeral shank (4) having, on the one hand, a diaphyseal stem (6) designed to be inserted into the cavity of a humerus and, on the other hand, an epiphyseal head (7) with a continuous cylindrical wall (8), of which the upper end defines a plane whose perpendicular forms an angle of 135° with the diaphyseal stem (6).

3. Inverted shoulder prosthesis (1) according to Claim 2, **characterized in that** the humeral implant (2) comprises a humeral insert (5) designed to be fixed to the epiphyseal head (7) and to receive the glenoid implant (3) in an articulated manner.

4. Inverted shoulder prosthesis (1) according to Claim 3, **characterized in that** the humeral insert (5) comprises a receiving surface (12) for the glenoid implant (3), said receiving surface (12) having the shape of a spherical cap.

5. Inverted shoulder prosthesis (1) according to Claim 3 or 4, **characterized in that** the humeral insert (5) comprises a cylindrical outer wall (9) which, at its lower end, has an annular collar (10) via which the humeral insert (5) is fixed to the humeral shank (4).

6. Inverted shoulder prosthesis (1) according to Claim 5, **characterized in that** the humeral insert (5) is fixed to the humeral shank (4) by a snap fit between a cylindrical fixing surface (11) of the insert (5), situated below the annular collar (10), and an inner surface of the cylindrical wall (8) of the epiphyseal head (7).

7. Inverted shoulder prosthesis (1) according to one of Claims 1 to 6, **characterized in that** the fixing member (16) of the glenoid sphere (15) comprises a male fixing cone (16), which is formed on a plane rear surface thereof and which is wedged in a female cone (17) formed in the base (14).

8. Inverted shoulder prosthesis (1) according to Claim 7, **characterized in that** the plane rear surface of the glenoid sphere (15) comprises an indexing member (20) designed to cooperate with a complementary indexing member (21) carried by the base (14), in such a way as to define the orientation of the off-centre of the sphere (15).

9. Inverted shoulder prosthesis (1) according to Claim 8, **characterized in that** the indexing member (20) is an orifice in which a peg (21), forming the complementary indexing member (21), is received.

10. Inverted shoulder prosthesis (1) according to one of Claims 7 to 9, **characterized in that** an orifice (18) designed to receive a screw (19) passes through the male fixing cone (16), and **in that** the female cone (17) comprises, in the continuation of its section for receiving the male cone (16), an internal thread designed to cooperate with the threading of the screw (19).
